# FASCICULE DE BREVET EUROPEEN

(11) **EP 0 966 532 B1**
(45) Date de publication et mention de la délivrance du brevet: **21.03.2007**
(21) Numéro de dépôt: 98903075.4
(22) Date de dépôt: 16.01.1998
(51) Int. Cl.: C12N 15/13, A61K 31/70, A61K 48/00, C12N 5/10

(54) **MATERIEL BIOLOGIQUE POUR LE TRAITEMENT D'UN MAMMIFERE PAR TRANSFERT DE GENE D'ANTICORPS ET COMPOSITION PHARMACEUTIQUE LE CONTENANT**
BIOLOGISCHES MATERIAL ZUR BEHANDLUNG EINES SÄUGETIERES DURCH TRANSFER EINESFÜR EINEN ANTIKÖRPER KODIERENDES GEN UND EINE PHARMAZEUTISCHE ZUSAMMENSETZUNGDIE DIESE ENTHÄLT
BIOLOGICAL MATERIAL FOR TREATING A MAMMAL BY ANTIBODY GENE TRANSFER AND PHARMACEUTICAL COMPOSITION CONTAINING SAME

(30) Priorité: 20.01.1997 FR 9700540
(43) Date de publication de la demande: 29.12.1999
(73) Titulaire: CENTRE NATIONAL DE LA RECHERCHE SCIENTIFIQUE (CNRS), 75016 Paris (FR)
(72) Inventeur: PIECHACZYK, Marc, F-34980 Saint Gély du Fesc (FR); NOEL, Danièle, F-34830 Clapiers (FR)
(74) Mandataire: Breese, Pierre
(86) Numéro de dépôt international: PCT/FR1998/000081
(87) Numéro de publication internationale: WO 1998/031808

(56) Documents cités:
- WO-A-90/06997
- FR-A- 2 706 486
- E. FENJVES ET AL.: "Systemic delivery of secreted protein by grafts of epidermal keratinocytes: Prospects for keratinocyte gene therapy." HUMAN GENE THERAPY, vol. 5, no. 10, octobre 1994, NEW YORK, NY, TATS-UNIS, pages 1241-1248, XP002044923 cité dans la demande
- K. KATO ET AL.: "Local production of the p40 subunit of interleukin 12 suppresses T-helper 1-mediated immune responses and prevents allogeneic myoblast rejection." PROCEEDINGS OF THE NATIONAL ACADEMY OF SCIENCES OF THE USA, vol. 93, no. 17, 20 août 1996, WASHINGTON, DC, TATS-UNIS, pages 9085-9089, XP002044924
- D. NOEL ET AL.: "Analysis of the individual contributions of immunoglobulin heavy and light chains to the binding of antigen using cell transfection and plasmon resonance analysis." JOURNAL OF IMMUNOLOGICAL METHODS, vol. 193, no. 2, 21 juin 1996, AMSTERDAM, PAYS BAS, pages 177-187, XP002044925
- R. BEERLI ET AL.: "Intracellular expression of single chain antibodies reverts ErbB-2 transformation." THE JOURNAL OF BIOLOGICAL CHEMISTRY, vol. 269, no. 39, 30 septembre 1994, BALTIMORE, MD, TATS-UNIS, pages 23931-23936, XP002044926
- S. AGER ET AL.: "Retroviral display of antibody fragments; Interdomain spacing strongly influences vector infectivity." HUMAN GENE THERAPY, vol. 7, no. 17, 10 novembre 1996, NEW YORK, NY, TATS-UNIS, pages 2157-2164, XP002044927
- D. NOEL ET AL.: "In vitro and in vivo secretion of cloned antibodies by genetically modified myogenic cells." HUMAN GENE THERAPY, vol. 8, no. 10, 1 juillet 1997, NEW YORK, NY, TATS-UNIS, pages 1219-1229, XP002044928
- NOEL D. ET AL: 'Skin as a potential organ for ectopic monoclonal antibody production' JOURNAL OF INVESTIGATIVE DERMATOLOGY vol. 118, no. 2, 2002, pages 288 - 294
- NOEL D. ET AL: 'High in vitro production of a model monoclonal antibody on adenoviral gene transfer' HUMAN GENE THERAPY vol. 13, 2002, pages 1483 - 1493
- PEREZ N. ET AL: 'Regulatable systemic production of monoclonal antibodies by in vivo muscle electroporation' GENETIC VACCINES AND THERAPY 23 Mars 2004, pages 1 - 5
- NOEL D. ET AL: 'Sustained systemic delivery of monoclonal antibodies by genetically modified skin fibroblasts' J INVEST DERMATOL vol. 115, 2000, pages 1 - 7

## Description

La présente invention concerne le domaine de la thérapie génique consistant à transférer dans les cellules d'un sujet au moins un gène codant pour une protéine thérapeutique. Plus particulièrement, l'invention concerne le transfert, dans des cellules ne produisant pas naturellement des anticorps, de séquences d'acide nucléique codant pour tout ou partie ou dérivé d'anticorps thérapeutiques impliquant une composante protéique participant à l'effet thérapeutique, de sorte que les cellules génétiquement modifiées par ces séquences d'acide nucléique et implantées chez un sujet produisent et sécrètent dans la circulation sanguine dudit sujet une quantité thérapeutiquement efficace de cet anticorps.

La thérapie génique consiste à corriger la déficience d'un gène en introduisant, dans les cellules où la déficience dudit gène est cause d'une pathologie, une séquence d'ADN portant l'information génétique permettant de remédier la déficiente. Les perspectives d'application de la thérapie génique dans le domaine des maladies génétiques sont nombreuses et l'on peut citer par exemple les corrections des thalassemies, de la drépanocytose, des déficits du métabolisme hépatique, de la mucoviscidose, des myopathies, etc ... (W. F. Anderson, 256, 808, 1992 ; R. C. Mulligan, Science, 260, 926, 1993 ; D. Miller, Nature, 357, 455, 1992 ; R. Morgan et W. F. Anderson, Ann. Rev. Biochem., 62, 191, 1993 ; B. Dodet, Biofutur, Mai 1992).

Mais la thérapie génique permet aussi de lutter contre des maladies qui ne relèvent pas exclusivement d'une déficience génétique, tels que des cancers ou des infections virales, en introduisant dans les cellules de l'organe ou du tissu atteint un gène codant pour une protéine ou un ARN thérapeutique. De telles substances thérapeutiques sont par exemple des cytokines, des anticorps intracellulaire, des variants de protéines virales, des ARNs antisens, des ribozymes, etc ....

Les techniques permettant l'introduction de l'information génétique dans des cellules sont décrites dans la littérature. Deux approches principales peuvent cependant être envisagées.

La première consistant à introduire la séquence d'ADN portant l'information génétique directement *in vivo* dans les cellules des organes ou tissus cibles de la thérapie ou dans des cellules d'organes ou de tissus chargés de produire la substance thérapeutique, soit au voisinage du lieu de production, soit de façon systémique.

La seconde, relevant de la thérapie cellulaire et dite *ex vivo,* consiste à prélever des cellules d'un sujet, à modifier ces cellules *in vitro* en y introduisant la séquence d'ADN portant l'information génétique que l'on souhaite transférer, puis à réintroduire les cellules ainsi modifiées dans l'organisme du sujet. Cette stratégie thérapeutique est par exemple décrite dans le brevet américain No. 5 399 346.

Les séquences d'ADN portant l'information génétique que l'on souhaite introduire dans les cellules sont associés fonctionnellement à des séquences d'ADN permettant leur expression *in vivo* et peuvent se présenter sous plusieurs formes :
- Dans le cas d'un transfert de gène directement *in vivo* selon la première approche envisagée ci-dessus, elles peuvent être utilisées sous forme :
   - libre, c'est à dire transférées sous forme d'ADN nu, comme un plasmide ou un fragment de restriction, notamment par injection *in vivo* dans les cellules, comme décrit dans la demande de brevet internationale publiée sous le numéro WO 90 11 092;
   - complexée ou associée à d'autres molécules favorisant leur entrée dans les cellules eucaryotes comme la lipofectin, le transfectace, le transfectam, la polyethylènimmine, etc...;
   - incorporée dans un vecteur viral, lequel sera introduit directement *in vivo* dans les cellules de l'organe ou du tissu cible par infection.
- Dans le cas d'un transfert de gène selon la seconde approche envisagée précédemment, dite *ex vivo*, la séquence d'ADN est intégrée *in vitro* dans des cellules qui sont ensuite introduites dans l'organisme du sujet; il peut s'agir alors par exemple de cellules souches hématopoïétique, de lymphocytes T, d'hépatocytes etc... Dans ce cas, les cellules génétiquement modifiées *in vitro* par la séquence d'ADN, selon les techniques décrites ci-dessus pour une introduction directement *in vivo*, peuvent avoir été prélevées du sujet traité ou provenir d'un autre sujet humain ou animal comme le porc (E. Cozzi et D. J. G. White, Nature Genetics, 1, 964-966, 1995).

Parmi, les substances capables d'interférer avec une pathologie et que l'on cherche à produire dans l'organisme du patient pour une thérapie génique, on peut citer cerains antigènes ou anticorps.

L'expression de séquences d'ADN codant pour des protéines antigéniques vise à permettre la production, par les cellules génétiquement modifiées par cet ADN, d'antigène susceptibles d'induire une immunisation de l'individu. Une telle stratégie de vaccination a par exemple été mise en oeuvre dans le cas de divers pathogènes dont le virus de la grippe (Tang, D., De Vit, M., et Johnston, Nature, 356, 152-154, 1992).

La production *in vitro* d'anticorps, de fragments d'anticorps ou de dérivés d'anticorps tels que des anticorps chimériques, par génie génétique dans des cellules eucaryotes a également déjà été décrite, par exemple dans les brevets européens publiés sous les numéros 120 694 et 125 023. L'injection à des patients d'anticorps thérapeutiques vise à cibler des antigènes impliqués dans une pathologie afin de neutraliser soit directement, soit par le biais d'une cascade d'événements métaboliques ou immunitaires, l'un des agents causals de la maladie. On peut citer comme exemples de telles stratégies thérapeutiques, le traitement ou la prévention de lymphomes B (Yefenof, E., Picker, L. I., Scheuermann, R. N., Vitetta, E. S., Street, N. E., Tucker, T., Uhr, J. W., Current Opinion in Immunology, 5, 740-744, 1993).

La demande de brevet internationale publiée sous le numéro WO 94 29 446 décrit l'expression intracellulaire de séquences d'ADN codant pour des anticorps. Cette approche permet d'envisager une thérapie génique directement *in vivo* de pathologie impliquant des composants cellulaires non accessibles par les méthodes de vaccination traditionnelles ou fondée sur la production *in vivo* d'antigènes recombinants. Les séquences d'ADN exprimées par les cellules génétiquement modifiées selon la méthode décrite dans la demande de brevet internationale WO 94 29 446 sont donc essentiellement caractérisées par le fait qu'elles comprennent un gène d'anticorps modifié de façon à ce que l'anticorps ne soit pas sécrété.

La présente invention vise au contraire à réaliser l'expression *in vivo* de gènes d'anticorps par des cellules qui sécréteront lesdits anticorps dans la circulation sanguine du mammifère porteur des cellules génétiquement modifiées par le gène d'anticorps.

Cette invention est fondée sur la mise en évidence que divers types cellulaires, autres que ceux produisant naturellement des anticorps sont capables, après modification génétique, de produire de manière stable *in vivo* des anticorps.

En effet, les plasmocytes, qui sont les cellules spécialisées pour la production d'anticorps, constituent de mauvais candidats pour la production à long terme d'anticorps thérapeutiques par transfert de gènes; les plasmocytes ont une durée de vie réduite, de l'ordre de quelques jours, et le fait qu'ils produisent déjà un autre anticorps est de nature à conduire à des associations ou des recombinaisons entre les chaînes de l'anticorps naturellement produit et l'anticorps exprimé par le gène transféré, ce qui est hautement préjudiciable à l'effet thérapeutique recherché. Il était donc important de montrer que des types cellulaires non spécialisés pour la production naturelle d'anticorps étaient susceptibles d'accepter un transfert de gène, d'exprimer in *vivo* un anticorps thérapeutique et de sécréter des niveaux soutenus avantageusement régulés d'anticorps dans la circulation sanguine d'un mammifère.

En conséquence, la présente invention concerne une composition pharmaceutique comprenant au moins une cellule de mammifère d'un type cellulaire ne produisant pas naturellement des anticorps, telle que les kératinocytes, les hépatocytes, les fibroblastes de la peau, les myoblastes, les cellules endothéliales et les cellules souches hématopoïétiques, génétiquement modifiée *in vitro* par une séquence d'acide nucléique, caractérisée en ce que ladite séquence d'acide nucléique contient un gène d'anticorps de type IgG et des éléments assurant l'expression *in vivo* dudit gène d'anticorps et la sécrétion dans la circulation sanguine d'un mammifère d'une quantité thérapeutiquement efficace de cet anticorps ou d'un fragment de celui-ci, par lesdites cellules génétiquement modifiées.

Par séquence d'acides nucléiques, on entend aussi bien des séquences d'ADN ou d'ARN ou des séquences contenant des nucléotides modifiés.

La séquence d'acides nucléiques entrant dans la composition de l'invention comprend :
- au moins un gène d'anticorps thérapeutique, c'est à dire un gène codant pour un anticorps natif non modifié donc naturel, ou un fragment d'anticorps, tels que les fragments Fab ou F(ab)'2 ou les fragments ScFv, ou encore un dérivé d'anticorps comme un anticorps chimérique ou un anticorps ou fragment d'anticorps fusionné à une substance effectrice par exemple une toxine ou une hormone;
- au moins un élément assurant l'expression du gène précédent; il s'agit de séquences promotrices de la transcription placées en amont du gène d'anticorps et contrôlant son expression dans les cellules ne produisant pas naturellement des anticorps.

Outre le gène d'anticorps et son promoteur, la séquence d'acides nucléiques peut comprendre une séquence de terminaison de la transcription, située en aval du gène d'anticorps et permettant la sécrétion du produit du gène d'anticorps dans la circulation sanguine du mammifère dont des cellules ont été génétiquement modifiées par la séquence d'acides nucléiques.

Le promoteur utilisé peut être tout promoteur permettant une expression efficace du gène qu'il contrôle dans le type cellulaire génétiquement modifié par la séquence d'acides nucléiques. Il peut ainsi être un promoteur viral, un promoteur ubiquitaire ou spécifique de tissu ou encore un promoteur synthétique.

La composition de l'invention est constituée de cellules ne produisent pas naturellement des anticorps, et se présentant sous une forme permettant leur incorporation dans l'organisme d'un mammifère ainsi qu'éventuellement leur culture préalable, lesdites cellules étant génétiquement modifiées par au moins une séquence d'acide nucléique contenant un gène d'anticorps de type IgG et des éléments assurant l'expression *in vivo* dudit gène d'anticorps et la sécrétion dans la circulation sanguine d'un mammifère d'une quantité thérapeutiquement efficace de cet anticorps ou d'un fragment de celui-ci.

Ce mode de réalisation peut être mis en oeuvre selon deux variantes:
- Soit, les cellules ne produisant pas naturellement des anticorps entrant dans la composition de l'invention proviennent du mammifère à traiter. Dans cette variante, les cellules sont préparées par les techniques consacrées de la biologie cellulaire et moléculaire, comme par exemple, à partir de biopsies prélevées du patient à traiter, puis ces cellules sont modifiées génétiquement par la séquence d'acide nucléique portant le gène d'anticorps, soit par transfection soit par infection par un vecteur conforme à ceux décrits précédemment dans le cas d'un transfert de gène directement *in vivo.* Les compositions pharmaceutiques fabriquées à partir de ce matériel biologique sont administrées en retour au patient dont les cellules ont été prélevées.
- Soit, les cellules ne produisant pas naturellement des anticorps entrant dans la composition de l'invention proviennent d'un autre mammifère humain ou animal que celui à traiter. Ces cellules ont été préparées comme dans la variante précédente. Dans le cas de cellules d'origine humaine, celles-ci proviennent de donneurs compatibles; dans le cas de cellules d'origine non-humaine, on utilise des cellules d'animaux génétiquement modifiées, comme le porc, rendues compatibles pour une greffe d'organe.

Les cellules précédentes se présentent sous une forme permettant leur implantation par tout moyen connu dans l'organisme du mammifère receveur. Elles peuvent en outre se présenter sous une forme ayant permis de les cultiver préalablement à la greffe. Il peut s'agir de tout support ou milieu de culture compatible avec leur adminsitration et leur incorpation chez le receveur, comme par exemple une matrice du type de celle décrite dans la demande de brevet européen publiée sous le numéro 378 576 concernant des fibroblastes.

On choisi des cellules ne produisant pas naturellement des anticorps mais possédant:
- la capacité de pouvoir sécréter des protéines dans la circulation sanguine d'un mammifère;
- une longue durée de vie dans l'organisme du mammifère, avantageusement d'au moins plusieurs mois à plusieurs années jusqu'à la vie entière du patient.

Plus particulièrement, ces cellules sont choisies pour leur capacité à accepter facilement d'être prélevées, modifiées génétiquement *ex vivo* et implantées chez un mammifère.

Parmi les type cellulaires présentant les caractéristiques précédentes, l'invention envisage plus spécifiquement les kératinocytes, les hépatocytes, les fibroblastes de peau, les myoblastes, les cellules endothéliales et les cellules souches hématopoïétiques.

Il a été démontré de manière surprenante (Fenjves, E. S., Smith, J., Zaradic, S., et Teichman, L. B., Human Gene Therapy, 5, 1241-1248, 1994) que les kératinocytes pouvaient produire relativement efficacement des protéines vers l'organisme et non pas seulement vers l'extérieur. En outre, leur culture est facile et en routine depuis plusieurs années dans les services hospitaliers pour les greffes de peau.

La manipulation des hépatocytes est plus difficile que celle des kératinocytes. Cependant, il a été montré (Grossman, M., Raper, S. E., Kozarsky, K., Stein, E. A., Engelhart, J. F., Müller, D., Lupien, P. J., Wilson, J. M., Nature Genetics, 6, 335-341, 1994 ; Ferry, N., Duplessis, O., Houssin, D., Danos, O., Heard J-M., Proc. Natl. Acad. Sci., USA, 88, 8377-8381, 1991) que les hépatocytes pouvaient être infectés par des rétrovirus recombinants à la fois *ex vivo* et *in vivo.*

La culture et la transduction rétrovirale des fibroblastes de peau sont faciles (Moullier, P., Maréchal, V., Danos, O, Heard, J-M., Transplantation, 56, 427-432, 1993). La manipulation des organoïdes est aisée (Moulier et al., Nature Genetics, 4, june 1993, 154-159). Les fibroblastes présentent l'avantage d'être facilement prélevable chez un sujet par une simple opération chirurgicale. En outre, des protocoles de thérapie génique sont en préparation pour la correction de déficits lysosomiaux chez les enfants.

Les myoblastes qui sont des cellules musculaires non différenciées, peuvent aussi être purifiés, et seront vraisemblablement utilisés sans modification génétique dans le cadre du traitement de certaines maladies dégénératives (Yao, S-N., Smith, K. J., et Kurachi, K., Gene Therapy, 1, 99-107, 1994).

La modification génétique de cellules endothéliales a déjà été réalisée pour produire des protéines thérapeutiques, par exemple dans la demande de brevet internationale PCT publiée sous le numéro WO 90 06997. Les cellules endothéliales, qui constituent la paroi des vaisseaux sanguins, sont donc particulièrement adaptées à la mise en oeuvre du matériel biologique de l'invention, dont le but est de faire sécréter, par les cellules génétiquement modifiées, les anticorps dans la circulation sanguine d'un mammifère.

D'autres types cellulaires peuvent être envisagés, telles que les cellules souches hématopoïétiques, dès lors qu'ils remplissent les caractéristiques définies plus haut.

La composition de l'invention trouve son application dans la préparation de compositions pharmaceutiques destinées à traiter ou à prévenir les rechutes de cancers, et les infections ou expansions virales plus particulièrement le SIDA.

Le cancer touche environ une personne sur quatre dans les populations occidentales et les traitements disponibles aujourd'hui ne sont réellement satisfaisants que pour un patient sur deux.

Des maladies virales graves touchent de manière de plus en plus importante les populations humaines, on pense bien entendu plus particulièrement aux virus HIV, pour lequel on ne dispose à l'heure actuelle d'aucun traitement efficace pour prévenir ou traiter l'infection.

Le matériel biologique de l'invention est remarquable en ce qu'il permet d'envisager une nouvelle approche thérapeutique de ces maladies très graves.

En effet, dans le cas des cancers, il permet à l'organisme de disposer sur le long terme d'anticorps spécifiques des cellules tumorales soit cytocides, soit induisant la dormance cellulaire. Ce but est atteint en utilisant des séquences d'acide nucléique portant un gène codant pour des anticorps dirigés contre un antigène spécifique de cellules tumorales.

Dans le cas des infections virales, le matériel biologique de l'invention permet à l'organisme de maintenir sur le long terme un niveau basal d'anticorps soit neutralisants pour les virus, soit cytocides pour les cellules infectées. Ce but est atteint en utilisant des séquences d'ADN codant pour des anticorps dirigés contre un antigène spécifique du virus responsable de la dite infection ou contre un antigène spécifique des cellules infectées par ledit virus.

La composition de l'invention peut contenir en outre des véhicules ou adjuvants classiquement utilisés. Les doses de cellules entrant dans ces compositions pharmaceutiques sont adaptées au mode d'administration utilisée, à la pathologie visée, de la séquence d'acide nucléique mise en oeuvre et de sa forme de présentation, pour permettre la production et la sécrétion d'une quantité thérapeutiquement efficace de l'anticorps dans la circulation sanguine du sujet traité.

Les cellules constituent un matériel biologique particulièrement adapté pour la préparation de compositions pharmaceutiques destinées à une thérapie cellulaire *ex vivo* d'un individu.

Les cellules humaines précédentes, dès lors qu'elles ne proviennent pas du patient chez lequel elles sont implantées, ou les cellules animales, sont bien entendu, préalablement à leur implantation, traitées par tous moyens physiques ou génétiques, connus de l'homme du métier, pour être protégées du système immunitaire du patient les recevant.

L'invention a encore pour objet l'utilisation de la composition pharmaceutique précédente, pour la préparation d'un médicament destiné au traitement des cancers ou d'infections virales.

Outre les caractéristiques qui précèdent, l'invention comporte d'autres caractéristiques qui apparaîtront au cours de la description qui suit et qui se réfèrent à des exemples expérimentaux de réalisation et de mise en oeuvre de la présente invention, étant entendu que ces exemples ne sauraient constituer une quelconque limitation à la portée des revendications.

Les travaux rapportés ci-dessous ont permis de démontrer que :
- *in vitro* des cellules prélevables chez le patient, modifiables génétiquement *ex vivo* et réimplantables, qui ne produisent pas naturellement des anticorps, sont capables de sécréter des anticorps recombinants conservant les propriétés de l'anticorps d'origine,
- au moins un type cellulaire précédent est capable de sécréter *in vivo* des anticorps recombinants conservant les propriétés de l'anticorps d'origine.
- le matériel biologique de l'invention n'induit dans l'organisme modifié aucune réponse immunitaire neutralisant l'anticorps recombinant.

### I - Définition de l'anticorps recombinant.

L'anticorps recombinant modèle utilisé pour les expériences de transfert de gène d'anticorps rapportées ci-après est un anticorps monoclonal de souris anti-thyroglobuline humaine (Tg10) (Piechaczyk et al., Hybridoma, vol. 4, 4, (1985), 361-367). Son clonage moléculaire et la caractérisation fonctionnelle des ADN complémentaires de sa chaîne lourde et de sa chaîne légère ont été effectués comme indiqué ci-après.

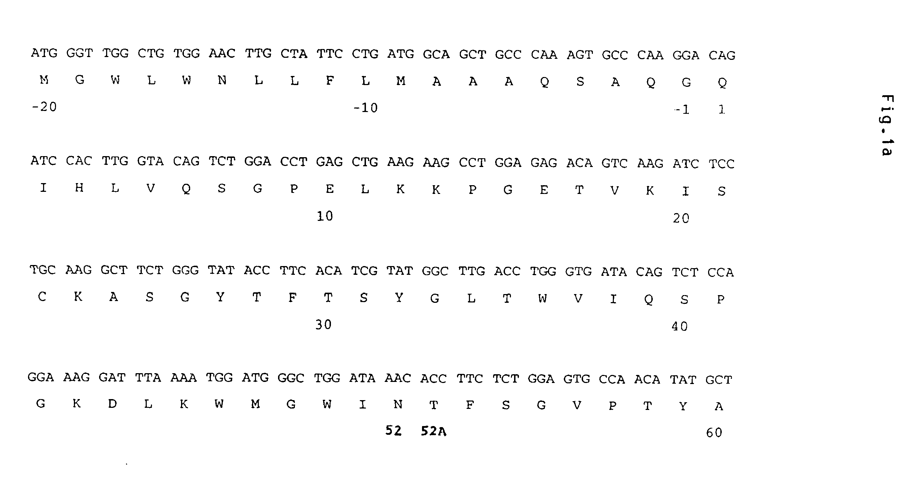

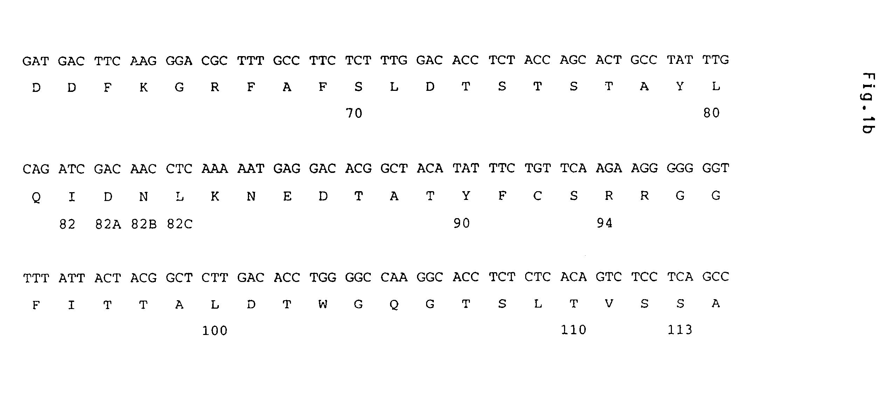

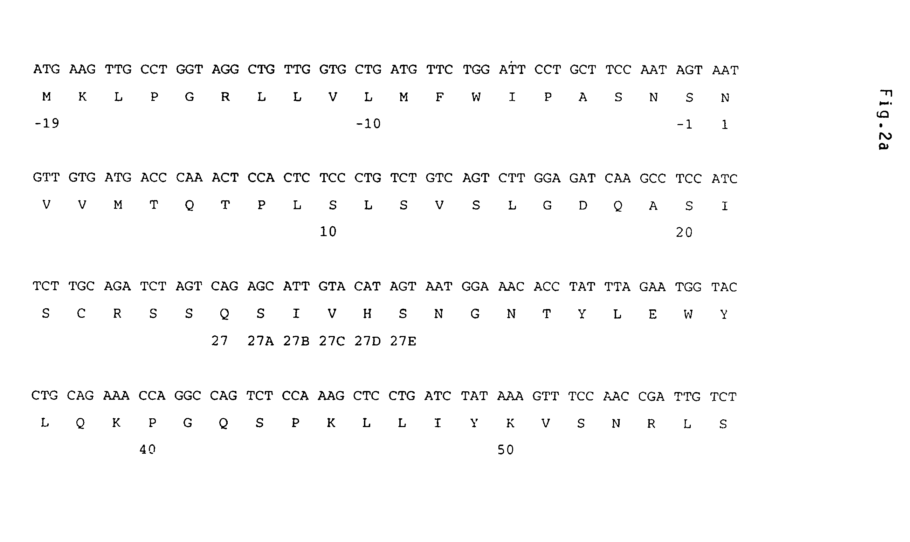

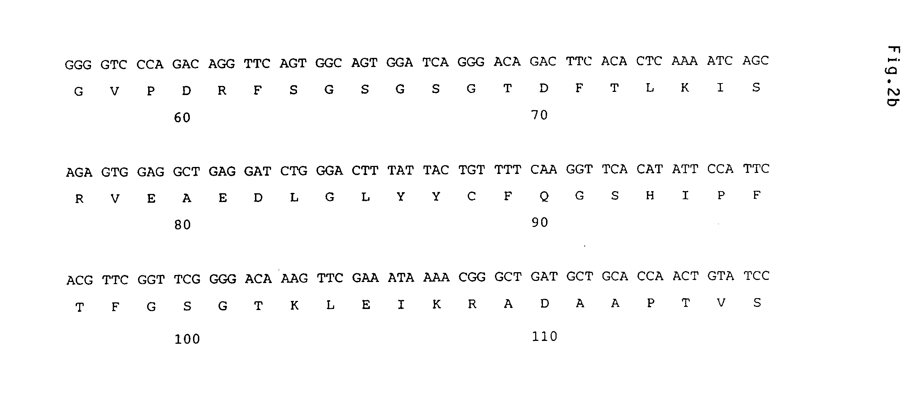

La thyroglobuline est une glycoprotéine iodée de haut poids moléculaire impliquée dans la synthèse, le stockage et la sécrétion des hormones thyroïdiques T3 et T4 (Marriq, C., C. Arnaud, M. Rolland, and S. Lissitzky. 1980. Eur. J. Biochem. 111:3347). Un anticorps monoclonal de souris, désigné ci-après Tg10, dirigé contre une région antigénique (région II) fréquemment reconnu par des autoanticorps naturels chez les patients atteints de maladie de Grave, de la thyroïdite de Hashimoto et des carcinomes de la thyroïde, a été établi par des membres du laboratoire CNRS UMR 9921 de la Faculté de pharmacie de Montpelllier, Avenue Charles Flahaut, 34060, Montpellier Cedex 01, France (Piechaczyk et al., Hybridoma, vol. 4, 4, (1985), 361-367). Les ADN complémentaires des chaînes légères (Kappa) et lourde (IgGl) de l'anticorps Tg10 ont été clonés dans le vecteur pSPORT1 (Gibco/BRL) par les techniques consacrées du génir génétique (Sambrook, J., E. F. Fritsch, and T. Maniatis. 1989. Molecular Cloning : A Laboratory Manual. Cold Spring Harbor Laboratory Press, Cold Spring Harbor, NY, USA). Les séquences nucléotidiques des parties variables des chaînes lourdes et légères qui spécifient chacune des chaînes d'anticorps ont été déterminées et sont représentées respectivement aux figures 1 et 2 en annexe. Le clonage et le séquençage des ADNc de l'anticorps Tg10 ont été effectués au laboratoire sus-mentionné.

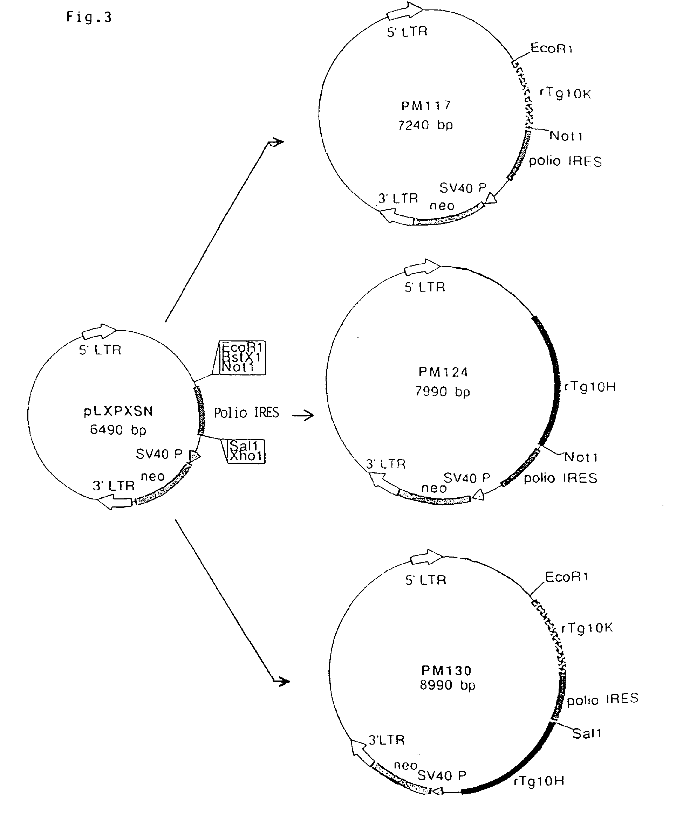

Pour leur caractérisation fonctionnelle, les ADNc de la chaîne légère et de la chaîne lourde de l'anticorps Tg10 ont été clonés dans le vecteur rétroviral pLXPXSN (Morgan, R. A., L. Couture, O. Elroy-Stein, J. Ragheb, B. Moss, and W. F. Anderson. 1992. Nucl. Acids Res 20:1293-1299) soit de part et d'autre de la séquence IRES du poliovirus endogène à ce vecteur pour former les vecteurs PM130, soit individuellement en amont de la séquence IRES pour former les vecteurs PM117 et PM124, comme réprésenté à la figure 3 en annexe). Les cellules simiennes COS-7 (ATCC CRL 1651) ont ensuite été transfectées par la technique au phosphate de calcium soit par PM130 seul, soit par la combinaison PM117+PM124. La présence dans les surnageants de culture d'anticorps réactifs contre la thyroglobuline humaine a été testée par la technique ELISA (Piechaczyk et al., Hybridoma, vol. 4, 4, (1985), 361-367). En outre, les constantes cinétiques d'association et de dissociation de l'anticorps recombinant Tg10 produit par les cellules COS-7 avec la thyroglobuline ont été déterminées à partir des surnageants de culture par résonance plasmonique de surface (Fagerstam, L. G., and R. Karlsson. 1993. Biosensor techniques. In Immunochemistry. V. Oss and M. vanRegenmortel, eds. M Dekker Inc. p.949-970) suivant la technique Biacore développée par la société Pharmaci Biosensor.

Les valeur de ces constantes sont rapportées dans le tableau 1 ci-dessous.

**Tableau 1**

| Anticorps | Constante cinétique d'association kₒₙ (M⁻¹s⁻¹) | Constante cinétique de dissociation k_{off} (s⁻¹) | Constante d'affinité Kₐ |
|---|---|---|---|
| Anticorps Tg10 naturel | 4,6 ± 0,1 x 10⁵ | 5,3 ± 0,2 x 10⁻⁵ | 8,6 ± 0,4 x 10⁹ |
| Anticorps Tg10 recombinant (PM117+PM124) | 1,4 ± 0,3 x 10⁵ | 4,3 ± 1,0 x 10⁻⁵ | 3,2 ± 1,4 x 10⁹ |
| Anticorps Tg10 recombinant (PM130) | 2,1 ± 1,5 x 10⁵ | 6,0 ± 0,4 x 10⁻⁵ | 3,5 ± 2,7 x 10⁹ |
| Chaîne lourde de l'anticorps Tg10 recombinant (PM117+PM124) | 1,0 ± 0,3 x 10⁵ | 3,0 ± 0,2 x 10⁻⁴ | 3.3 ± 1,2 x 10⁸ |

De façon surprenante, le tableau 1 montre que la chaîne lourde synthétisée seule à partir de PM124 est sécrétée par les cellules COS-7 et reconnaît la thyroglobuline humaine avec une affinité diminuée seulement de 10 fois par rapport à l'anticorps complet.

### II - Lignées productrices de rétrovirus.

La plupart des cellules primaires sont extrêmement sensibles aux méthodes de transfection classiques. En outre, la durée de vie, et donc l'expression, de l'ADN transfecté est en général très courte dans la plupart des cellules transfectées.

Pour permettre une infection efficace de types cellulaires variés et une expression sur le long terme de l'anticorps Tg10 dans les cellules génétiquement modifiées, une lignée cellulaire productrice de rétrovirus recombinants véhiculant et exprimant les ADNc de l'anticorps Tg10 a été établie.

Les cellules d'empaquetage rétroviral amphotrope PA 317 (Miller, D. and Buttimore, 1986, Molec. Cell Biol. 6, 2895-2902) ont été transfectées par la technique du précipité au phosphate de calcium par le vecteur rétroviral PM130. Plusieurs clones producteurs stables ont été établis. La lignée PA130.10 a été utilisée pour les expériences d'infection ultérieures. Son titre en virus, dosé sur la lignée indicatrice NIH 3T3 (Miller, D. and Buttimore, 1986, Molec. Cell Biol. 6, 2895-2902) a été de 10⁴ cfu/ml.

### III - Expériences in vitro.

Les rétrovirus produits par la lignée PA130.10 ont été utilisés pour infecter différentes lignées cellulaires établies représentatives de différents types cellulaires disponibles auprès de l'American Type Culture Collection (ATCC) :
- lignée NIH3T3 de fibroblastes murins;
- lignée A431 de kératinocytes humains;
- lignée HepG2 d'hépatocytes humains;
- lignée C2C12 de myoblastes.

Différents clones cellulaires ont été dérivés pour chaque type de transduction rétrovirale et l'anticorps Tg10 produit dans le surnageant de culture a été dosé par ELISA. Les résultats obtenus sont rapportés dans le tableau 2 ci-dessous.

**Tableau 2**

| Lignées | Anticorps Tg10 |
|---|---|
| Lignée NIH3T3 | 88 +/- 65 ng / 10⁵ cellules / 24hrs |
| Lignée A431 | 35 +/- 6 ng / 10⁵ cellules / 24hrs |
| Lignée HepG2 | 3,5 +/- 1,5 ng / 10⁵ cellules / 24hrs |
| Lignée C2C12 | 2 +/- 0,6 ng / 10⁵ cellules / 24hrs |

En outre, dans le cas des myoblastes C2C12 différenciés in vitro en myotubes la production est conservée.

Les propriétés thermodynamiques et cinétiques des anticorps produits par ces différents types cellulaires déterminées par résonance plasmonique de surface selon la technogie BIAcore (Pharmacia Biosensor) se sont révélées être identiques à celles de l'anticorps Tg10 de départ.

Les vecteurs rétroviraux ont dans un second temps été utilisés pour infecter des fibroblastes primaires de peau de souris (infection rétrovirale et des hépatocytes humains (transfection). Les productions en anticorps ont été respectivement de :
- 10 à 20 ng / 10⁵ cellules / 3 jours, et de
- 1 à 10 ng / 10⁵ cellules / 4 jours.

De même, les caractéristiques des anticorps produits ont été les mêmes que celles de l'anticorps de départ.

### IV - Expérience in vivo.

Des cellules C2C12 modifiées génétiquement et qui ont conservé la capacité de se différencier en myotubes ont été implantées par injection dans les jambiers antérieurs de 4 souris syngéniques C3H à raison de 10⁷ cellules par jambier.

Chez 3 des 4 souris, la production d'anticorps recombinants ayant conservé les propriétés thermodynamique et la propriété de reconnaissance de l'antigène de l'anticorps de départ a été suivie pendant deux mois. La quantité d'anticorps produite s'est régulièrement élevée du niveau de base à une production d'environ 100 ng/ml de sérum.

### V - Absence de réponse immunitaire neutralisant l'anticorps recombinant.

Un des buts essentiels de l'invention est de faire produire de manière systémique un anticorps recombinant, avantageusement thérapeutique par des cellules génétiquement modifiées de mammifère.

Un risque possible de cette approche est l'induction d'une réponse immunitaire de la part de l'organisme modifié pouvant entrainer la neutralisation de l'anticorps recombinant.

Cette objection a été écartée par les résultats expérimentaux présentés ci-dessous.

2 x 10⁷ cellules myogèniques primaires exprimant de façon stable l'anticorps monoclonal Tg10 après transduction rétrovirale sont implantées au niveau du *tibialis anterior* de souris C3H. Le sérum des souris est prélevé à des intervalles d'une semaine pendant plusieurs mois. La quantité d'anticorps Tg10 sécrétés est dosée par la méthode ELISA. En parallèle, la quantité d'anticorps anti-idiotype est déterminée par ELISA.

Dans une série de 5 souris, la sécrétion de l'anticorps Tg10 s'est située entre 100 et 300 ng/ml de sérum pendant 4 mois. Aucune réponse anti-idiotype n'a pu être détectée dans ces conditions.

## Revendications

1. Composition pharmaceutique, comprenant au moins une cellule de mammifère d'un type cellulaire ne produisant pas naturellement des anticorps, telle que les kératinocytes, les hépatocytes, les fibroblastes de la peau, les myoblastes, les cellules endothéliales et les cellules souches hématopoïétiques, génétiquement modifiée *in vitro* par une séquence d'acide nucléique, **caractérisée en ce que** ladite séquence d'acide nucléique contient un gène d'anticorps de type IgG et des éléments assurant l'expression *in vivo* dudit gène d'anticorps et la sécrétion dans la circulation sanguine d'un mammifère d'une quantité thérapeutiquement efficace de cet anticorps ou d'un fragment de celui-ci, par lesdites cellules génétiquement modifiées.

2. Composition pharmaceutique selon la revendication 1, **caractérisée en ce que** les cellules ne produisant pas naturellement des anticorps proviennent soit du mammifère à traiter, soit d'un autre mammifère que celui à traiter ayant subi un traitement les rendant compatibles.

3. Composition pharmaceutique selon l'une quelconque des revendications précédentes, **caractérisée en ce que** les cellules ne produisant pas naturellement des anticorps sont choisies parmi celles possédant :
- la capacité de pouvoir sécréter des protéines dans la circulation sanguine d'un mammifère ;
- une longue durée de vie dans l'organisme d'un mammifère, d'au moins plusieurs mois à plusieurs années jusqu'à la vie entière du patient.

4. Composition pharmaceutique selon l'une quelconque des revendications précédentes, **caractérisée en ce que** le gène d'anticorps est un gène codant pour un anticorps natif, un fragment ou un dérivé de cet anticorps tel qu'un anticorps chimérique.

5. Composition pharmaceutique selon la revendication 4, **caractérisée en ce que** ledit anticorps, fragment ou dérivé d'anticorps est dirigé contre un antigène spécifique de cellules tumorales.

6. Composition pharmaceutique selon l'une quelconque des revendications 1 à 5 comprenant un véhicule pharmaceutiquement acceptable.

7. Utilisation d'une composition pharmaceutique selon l'une quelconque des revendications 1 à 5, pour la préparation d'un médicament destiné au traitement des cancers ou d'infections virales.

## Claims

1. Pharmaceutical composition, comprising at least one cellular type mammal cell that does not naturally produce any antibodies such as keratocytes, hepatic cells, fibroblasts of the skin, myoblasts, endothelial cells and haematopoietic stem cells genetically modified *in vitro* by a nucleic acid sequence, **characterised in that** said nucleic acid sequence contains an IgG type antibody gene and elements for *in vivo* expression of said antibody gene and secretion of a therapeutically efficient quantity of this antibody or a fragment of this antibody in the blood circulation of a mammal, by said genetically modified cells.

2. Pharmaceutical composition according to claim 1, **characterised in that** the cells that do not naturally produce antibodies originate either from the mammal to be treated or from a different mammal that has received a treatment to make it compatible with the mammal to be treated.

3. Pharmaceutical composition according to any of the previous claims, **characterised in that** the cells that do not naturally produce antibodies are chosen from among cells with:
- the ability to secrete proteins into the blood circulation of a mammal;
- a long life in the organism of a mammal, varying from at least several months to several years, and up to the entire life of the patient.

4. Pharmaceutical composition according to any of the previous claims, **characterised in that** the antibody gene is a coding gene for a native antibody, a fragment or a derivative of this antibody such as a chimeric antibody.

5. Pharmaceutical composition according to claim 4, **characterised in that** said antibody, fragment or a derivative of an antibody is directed against an antigen specific to tumour cells.

6. Pharmaceutical composition according to any of claims 1 to 5, comprising a pharmaceutically acceptable vehicle.

7. Use of a pharmaceutical composition according to any of claims 1 to 5, for preparation of a medication for the treatment of cancers or viral infections.

## Patentansprüche

1. Pharmazeutische Zusammensetzung, mindestens eine Säugetierzelle eines Zelltyps enthaltend, der keine Antikörper natürlich produziert, wie die Keratinozyten, die Hepatozyten, die Fibroblasten der Haut, die Myoblasten, die Endothelzellen und die hämatopoetischen Stammzellen, genetisch modifiziert *in vitro* durch eine Nukleinsäuresequenz, **dadurch gekennzeichnet, dass** die besagte Nukleinsäuresequenz ein Antikörpergen vom Typ IgG und Elemente enthält, die die Expression *in vivo* des besagten Antikörpergens und die Sekretion in den Blutkreislauf eines Säugetiers einer therapeutisch wirksamen Menge dieses Antikörpers oder eines Fragments desselben gewährleisten, durch die besagten genetisch modifizierten Zellen.

2. Pharmazeutische Zusammensetzung nach Anspruch 1, **dadurch gekennzeichnet, dass** die Zellen, die keine Antikörper natürlich produzieren, entweder aus dem zu behandelnden Säugetier oder aus einem anderen Säugetier als dem zu behandelnden Säugetier hervorgehen, das eine Behandlung absolviert hat, um kompatibel zu werden.

3. Pharmazeutische Zusammensetzung nach einem der vorhergehenden Ansprüche, **dadurch gekennzeichnet, dass** die Zellen, die keine Antikörper natürlich produzieren, aus denen ausgewählt sind, welche besitzen:
- die Fähigkeit zur Sekretion von Proteinen in den Blutkreislauf eines Säugetiers,
- eine lange Lebensdauer im Organismus eines Säugetiers von mindestens mehreren Monaten bis mehreren Jahren bis zum gesamten Leben des Patienten.

4. Pharmazeutische Zusammensetzung nach einem der vorhergehenden Ansprüche, **dadurch gekennzeichnet, dass** das Antikörpergen ein für einen nativen Antikörper kodierendes Gen ist, ein Fragment oder ein Derivat dieses Antikörpers wie ein chimerischer Antikörper.

5. Pharmazeutische Zusammensetzung nach Anspruch 4, **dadurch gekennzeichnet, dass** der besagten Antikörper, das Fragment oder das Antikörperderivat gegen ein spezielles Antigen von Tumorzellen gerichtet ist.

6. Pharmazeutische Zusammensetzung nach einem der Ansprüche 1 bis 5, einen pharmazeutisch akzeptablen Überträger umfassend.

7. Verwendung einer pharmazeutischen Zusammensetzung nach einem der Ansprüche 1 bis 5 für die Zubereitung eines Medikaments, das zur Behandlung von Krebserkrankungen oder Virusinfektionen bestimmt ist.
